# EUROPEAN PATENT SPECIFICATION

(11) **EP 2 939 645 B1**
(45) Date of publication and mention of the grant of the patent: **21.10.2020**
(21) Application number: 15165909.1
(22) Date of filing: 30.04.2015
(51) Int. Cl.: A61G 10/04, A61B 5/083, A61M 16/12, A61B 5/087

(54) **APPARATUS FOR THE DETECTION OF THE TOTAL AMOUNT OF CARBON DIOXIDE CONTAINED IN A FLOW OF GAS**
VORRICHTUNG ZUR BESTIMMUNG DER GESAMTMENGE VON KOHLENDIOXID IN EINEM GASSTROM
APPAREIL POUR LA DÉTECTION DE LA QUANTITÉ TOTALE DE DIOXYDE DE CARBONE DANS UN FLUX DE GAZ

(30) Priority: 30.04.2014 IT MI20140802
(43) Date of publication of application: 04.11.2015
(73) Proprietor: Dimar S.R.L. Unipersonale, 41036 Medolla (MO) (IT)
(72) Inventor: Borsari, Maurizio, 41037 MIRANDOLA (MO) (IT)
(74) Representative: Branca, Emanuela

(56) References cited:
- US-A- 5 335 653
- US-A- 5 850 833
- US-A1- 2002 173 728
- US-A1- 2009 118 633
- US-A1- 2012 265 089
- US-B1- 6 534 769

## Description

The present invention relates to an apparatus for detecting the total amount of carbon dioxide per unit of time contained in a flow exhaled by a patient.

The present invention is suitable for use in health, medical and hospital fields in which it is necessary, depending on the pathologies that affect patients, to periodically monitor the amount of carbon dioxide eliminated by the same.

As is known, patients in conditions of hypoxia, cyanosis, dyspnea, orthopnea, gasping, abnormal respiratory rate, altered state of consciousness and/or subject to pathological respiratory sounds such as, for example, rales, rhonchi, puffs and/or whistling, inevitably require the administration of oxygen that can be effected with the use of various means such as, for example, nasal cannulas, simple face masks, Venturi masks, nasopharyngeal catheters, transtracheal catheters, endotracheal tubes or cannulas, NIMV (Non-invasive mechanical ventilation), CPAP (continuous pressure ventilations in the air passages) and/or the like.

The continuation of any respiratory problem that does not allow an adequate elimination or expulsion of carbon dioxide from the organism can cause a consequent increase in the concentration of carbon dioxide in the blood. This phenomenon, called hypercapnia, can have serious consequences for the patient, exerting a depressive effect on the central nervous system, with headaches, confusion and even coma, that can lead to death due to hypercapnia.

It is therefore of fundamental importance to monitor the capacity that patients affected by respiratory problems have in expelling carbon dioxide.

The means used and techniques implemented for both monitoring a patient's conditions and for an improvement in the same, naturally vary from case to case, also depending on the contingent situation.

Capnometry is known among the various techniques used for having an indication on the degree of impairment of the respiratory functions, whereby the partial pressure of carbon dioxide present in the air exhaled by the patient is measured and registered, measuring the above-mentioned value of carbon dioxide on the final part of each single expiratory act.

This measurement, called ETCO₂ (end-tidal CO₂) in the field, is the only non-invasive solution currently usable for revealing the percentage of carbon dioxide exhaled by a patient with time.

With capnometry, it is possible to reveal possible reductions in the ETCO₂ due to various kinds of pathological states, in both intensive care and also during ventilation of the patients. This technique can be applied to intubated or tracheotomized patients, as on spontaneously breathing patients with the use of appropriate measurement instruments, called capnographs, which exploit emission electromagnetic radiation optical sensors within the infrared frequency range.

Although the measurement of the ETCO₂, as currently effected, allows the patient's state to be monitored through the monitoring of the concentration of the carbon dioxide exhaled, it has various significant limitations in terms of flexibility of use, as the traditional measurement of the ETCO₂ cannot be effected in the presence of respiratory interfaces, such as, for example, face masks, NIMV helmets or any other similar medical device.

This limit is, on the one hand, due to the difficulty in coupling the sensors of the capnograph with these respiration interfaces, and on the other, to the difficulties in maintaining a correct position of the sensors during the treatment, which must exclusively reveal the gas exhaled by the patient, in addition to the high flow quantities used.

The erroneous position of the sensors of the capnographs inside the above respiratory interfaces, and the high amount of flow used, lead to a dilution in the concentration of carbon dioxide exhaled, significantly falsifying the final values revealed which are unreliable and unusable.

Consequently, the use of traditional capnographs during assisted NIMV ventilation treatments does not allow certain data to be obtained. The risk of dilutions of the gases exhaled by the patient with the gases introduced into the respiratory interface is so high that the results revealed by these measurements are extremely unreliable and uncertain.

Furthermore, the ETCO₂ measurement does not represent a measurement of the concentration and/or actual content of the carbon dioxide in the flow of gas exhaled per unit of time, but it only represents the extrapolation of this value on the basis of the measurement of the sole level of carbon dioxide in the final part of the exhalation. With the measurement of the ETCO₂, it is therefore not possible to obtain continuous information on the real trend of the concentration and/or content of carbon dioxide in the gas exhaled by the patient. This clearly represents a limit when there are anomalies in the patient's respiration that the ETCO₂ measurement is not capable of revealing, which, in turn, can lead to a worsening in the patient's possible condition due to the lack of a timely intervention on the part of the personnel.

As it is therefore not possible to effect the traditional measurement of ETCO₂ during the use of known respiration interfaces, the patient must be periodically subjected to arterial blood gas analysis, called EGA or Emogas, through which the amount of oxygen and carbon dioxide present in the patient's blood can be measured.

It is also possible to resort to blood gas analysis for effecting all the regulation and calibration operations of the devices necessary for implementing the respiratory support and oxygenation treatment and for verifying whether these regulations have caused an improvement in the clinical conditions of the patient.

As the amount of carbon dioxide dissolved in the blood varies very slowly with time, in order to know the amount of carbon dioxide eliminated by the patient and his physiological conditions, blood gas analysis must be effected periodically, generally one every eight hours and, in serious cases or in the case of emergency, one every hour.

It should be pointed out, however, that blood gas analysis requires arterial blood sampling which is extremely delicate and complex. Blood gas analysis, in fact, is normally effected in intensive care units, such as, for example, CPR, first aid or the like.

Very rarely and only in the case of extreme necessity is blood gas analysis effected in other hospital departments as it is complicated and painful, and consequently an excessive accumulation of carbon dioxide in the blood is normally revealed by observing the variations in the skin colouring of the patient with time, his respiratory rate and other clinical parameters that can lead to suspecting an increase in the carbon dioxide content in the blood. Mutations in these clinical parameters, however, indicate an advanced degenerative state which inevitably requires an emergency intervention and transferring the patient to an intensive care unit, with further risks of infection and a considerable increase in the overall costs for the handling and treatment of the same.

The most reliable method for evaluating the blood pressure of carbon dioxide (PaCO₂) present in the blood is therefore EGA. EGA, however, when effected, is not capable of providing a continuous track of the PaCO₂ trend but only of providing single values obtained from intermittent measurements effected at discrete time intervals.

Bearing in mind that the parameters of the ventilatory function of the patient can vary rapidly without evident alterations in the overall clinical state of the same, the specific choice of effecting samplings is particularly complicated and depends exclusively on the experience and sensations of the doctors on duty.

The estimation of the PaCO₂ can also be effected using another non-invasive monitoring technique, i.e. transcutaneous measurement. According to this technique, the PaCO₂ of the patient is obtained from the measurement of the transcutaneous carbon dioxide, i.e. that relating to the capillary blood flow of the tissues underlying an area of the body which is suitably heated.

The transcutaneous measurement, however, requires a high preparation level of the medical/hospital staff, a considerable consumption of materials linked to the frequent substitutions and recalibrations of the sensors used (during the transcutaneous measurement, the sensors used must be periodically moved to avoid burning the involved portion of the patient's skin), lengthy actuation times, in addition to high management and operating costs.

It should finally be pointed out that the accuracy of the data measured is particularly variable from one skin area to another, significantly limiting the measurement only to certain specific areas of the body.

Again with reference to EGA, this is also used for monitoring the state of patients during extracorporeal circulation "EC" techniques, called ECMO or DECAP and/or the like, used in intensive care for treating patients with heart failure and/or severe acute respiratory insufficiency.

In particular, ECMO or DECAP consists in causing the blood to circulate through a blood/gas exchanger, consisting of a disposable membrane permeable to gases and in correspondence with which there is an exchange of oxygen and carbon dioxide towards and from the blood.

As ECMO/DECAP is a continuative daily treatment, which can also last for 30 days, the ongoing work of the membrane can cause a reduction in the effectiveness of the same, which requires, on the one hand, the immediate variation in the overall setting parameters of the system used, and on the other, the possible substitution of the membrane with a new higher performing membrane.

Although EGA allows the amount of carbon dioxide dissolved in the blood to be revealed, the lengthy time intervals between each sampling to be analyzed do not allow an immediate identification of a drop in the performances of the exchange membrane, whose reduced effectiveness with time inevitably causes an excessive accumulation of carbon dioxide in the patient's blood with serious consequences for the same.

For the same reason, also the setting of the apparatus, especially in the initial phase of the treatment, is not facilitated in rapid times by blood gas analysis as it is managed by the doctor on duty who, on the basis of his experience, establishes the parameters to be set at the beginning of or during the treatment.

It should also be added that, at present, it is not possible to continuously know the amount of carbon dioxide eliminated by the patient during extracorporeal circulation "EC" treatments (ECMO/DECAP), with the consequent need to refer to the values of carbon dioxide dissolved in the blood revealed by means of EGA.

It is therefore evident that the necessity is strongly felt in the medical field for an apparatus and process for the high-reliability monitoring of the trend of the concentration and/or amount of carbon dioxide in the gas exhaled and in a patient's blood in real time, which overcomes the drawbacks of the known art and which, at the same time, is simple to use and economically advantageous.

American patent US 4,856,531 (in the name of Meriläinen) describes an apparatus for monitoring the release of carbon dioxide, the oxygen consumption and respiration quotient of a patient connected to a respirator. The apparatus measures the above parameters suitably mixing, in a mixing chamber, the gas exhaled by the patient with a flow of air and measuring the ETCO₂ (end-tidal) of the mixed flow in time points by means of intermittent withdrawals by sampling of a fraction of the mixed flow.

Patent US 5,335,653 (in the name of Blomqvist et al.) describes a method and apparatus for supplying a patient with a stable mixture of gas. The apparatus also comprises a measurement unit of the gas exhaled by the patient. As can be seen in column 4, lines 51-61, this is the same ETCO₂ measurement method as patent US 4,856,531 discussed above, which therefore envisages intermittent sampling withdrawals from the flow of gas exhaled mixed with another gaseous flow.

Finally, patent application US 2012/0265089 (in the name of Orr) describes the determination of respiration parameters of a subject starting from a diluted flow of exhaled gas. Also in this case, the measurement is effected by sampling using an interface of the ETCO₂ type. Furthermore, there are high losses in this method.

The main objective of the present invention is therefore to propose an apparatus for continuously detecting, in real time and per unit of time, the actual concentration and amount of carbon dioxide, total and not sampled, released by a patient during a certain time period, eliminated by a patient by exhalation in a certain time period inside a respiration interface.

An objective of the present invention is to propose a high-reliability apparatus for detecting the actual concentration and/or amount of carbon dioxide, in real time, total and not sampled, per unit of time, in a flow of gas to be monitored, which overcomes the drawbacks of the known art described above.

Another objective of the present invention is to ensure the correct detection of the concentration and total amount of carbon dioxide eliminated by a patient during a non-invasive ventilation treatments.

Yet another objective of the present invention is to facilitate the setting, in useful time, of the parameters to be established during the non-invasive ventilation treatments.

A further objective of the present invention is to avoid the excessive or delayed use of blood gas analysis.

Another objective of the present invention is to ensure an optimal management of and solution to problems relating to Acute Respiratory Insufficiency (ARI) during NIMV treatments, when said problems are of a hypoxemic nature and also when they are of a hypercapnic nature.

An additional objective of the present invention is to significantly reduce the overall hospitalization costs linked to the management and treatment of patients affected by the above respiratory illnesses.

A further objective of the present invention is also to avoid or reduce the number of hospitalizations of patients affected by mild respiratory problems in intensive care units due to an aggravation of the same as a result of the onset of non-monitored hypercapnia.

Yet another objective of the present invention is to ensure the monitoring of the amount of carbon dioxide eliminated from patients' blood during extracorporeal circulation "EC" treatment (ECMO/DECAP).

An objective of the present invention is also to provide a rapid and immediate indication of the drop in effectiveness of the exchange membranes used in the extracorporeal circulation "EC" treatment (ECMO/DECAP).

Another objective of the present invention is to facilitate the setting of the parameters to be established during the extracorporeal circulation "EC" treatment (ECMO/DECAP).

The objectives specified above and others, are achieved by an apparatus for detecting the total amount of carbon dioxide per unit of time contained in a flow of gas to be monitored, as defined in claim 1. The dependent claims define further preferred embodiments.

A description is now provided for illustrative purposes, of a preferred but non-exclusive embodiment of a process and apparatus for detecting the actual concentration of carbon dioxide per unit of time contained in a flow of gas to be monitored, according to the present invention.

The description is effected hereunder with reference to the enclosed drawings, provided for purely indicative and therefore non-limiting purposes, wherein:
- figure 1 is a block scheme of the functioning of an apparatus and process for detecting the concentration and/or amount of carbon dioxide per unit of time contained in a flow of gas to be monitored, according to the present invention;
- figure 2 is a schematic representation of a respiration interface, in particular a helmet, for assisted respiration (NIMV) that can be used in relation to the present process;
- figure 3 is a schematic representation of an exchange membrane in use in extracorporeal oxygenation "EC" treatment (ECMO/DECAP) that can be used in relation to the present process;
- figure 4 is a general block scheme of an apparatus for detecting the concentration of carbon dioxide released by a patient into a chamber provided with an inlet and an outlet, according to the present invention;
- figure 5 is a detailed block scheme of the apparatus according to figure 4;
- figure 6 is a schematic representation of the apparatus according to figures 4 and 5.

The present invention relates to a process P for the real-time and high-reliability detection E of the actual concentration and/or total amount Q of carbon dioxide 2 per unit of time contained in a flow of gas to be monitored X.

It should be pointed out that the term "real time" is intended to express the immediacy of the detection of the concentration and/or amount Q of carbon dioxide contained in the flow of gas to be monitored X, whereas the term "actual concentration and/or amount" refers to the effective concentration and/or amount of carbon dioxide, total and not sampled, contained in the flow of gas to be monitored at each moment in which the detection is effected without the need for making estimates. By measuring the concentration and/or amount of carbon dioxide in the whole flow and not in a sampling thereof, we obtain the total value, with an approximation as much precise as the greater the frequency of the detection is.

It should also be pointed out that, in the present description, each flow of gas mentioned refers to a respective fluid composed of a mixture of gases, whose composition and concentrations of the gases contained therein can be known, or they must be revealed, measured and extrapolated.

More specifically, the process P comprises a mixing phase of the flow of gases to be monitored X with a first flow of gas 4 which comprises a known concentration and/or amount of carbon dioxide 2 thus obtaining a second flow of gas 5.

The process P also comprises a continuous measuring phase of the concentration and/or amount of carbon dioxide 2 contained in the second flow of gas 5, at regular time intervals, and a continuous measuring phase of the rate of the first and/or second flow of gas 4, 5.

This is also followed by a phase F of continuously determining the concentration and/or amount Q of carbon dioxide 2 per unit of time, contained in the flow of gas to be monitored X, and a phase of continuously visualizing, preferably by means of at least one display 403 or a similar visualization device, the concentration and/or amount Q of carbon dioxide 2 per unit of time, of the flow of gas to be monitored X.

The mixing of the first flow of gas 4 with the flow of gas to be monitored X containing carbon dioxide, advantageously takes place in a chamber 1 provided with an inlet 1a of the first flow of gas 4, a further inlet of the flow of gas to be monitored X, and an outlet 1b of said second flow of gas 5.

The known concentration and/or amount of carbon dioxide 2 of the first flow 4 is also advantageously determined by direct measurement.

The measurement of the concentration and/or amount of carbon dioxide 2 in the first and second flows of gas 4, 5 or in the sole second flow of gas 5 is preferably carried out by means of one or more optical detectors 6, advantageously infrared detectors.

According to a preferred aspect of the present invention, the measurement of the concentration and/or amount of carbon dioxide 2 in the first and second flows of gas 4, 5 or in the sole second flow of gas 5 is carried out at a frequency ranging from 1 Hz to 120 Hz, preferably from 80 Hz to 120 Hz, even more preferably 100 Hz.

The measurement of the concentration and/or amount of carbon dioxide 2 in the first and second flow of gas 4, 5 or in the sole second flow of gas 5 by means of the detectors 6 is advantageously carried out in parts per million.

According to a preferred aspect of the present invention, the measurement of the rate is carried out in the first and/or second flow of gas 4, 5, preferably by means of at least one or more flow meter.

According to an advantageous aspect of the present invention, the step of determining F the concentration and/or amount of carbon dioxide 2 per unit of time is carried out by means of an algorithm correlating the concentration and/or amount of carbon dioxide 2 measured in the second flow of gas 5 and the rate of the second flow of gas 5 with the known concentration and/or amount of carbon dioxide 2 of the first flow of gas 4 to provide a measurement of the amount Q of carbon dioxide 2 in said flow of gas to be monitored X in a desired unit of time.

In particular, the concentration and/or amount of carbon dioxide 2 in the flow of gas to be monitored X per unit of time can be expressed in milliliters or liters per minute and/or in millimeters of mercury.

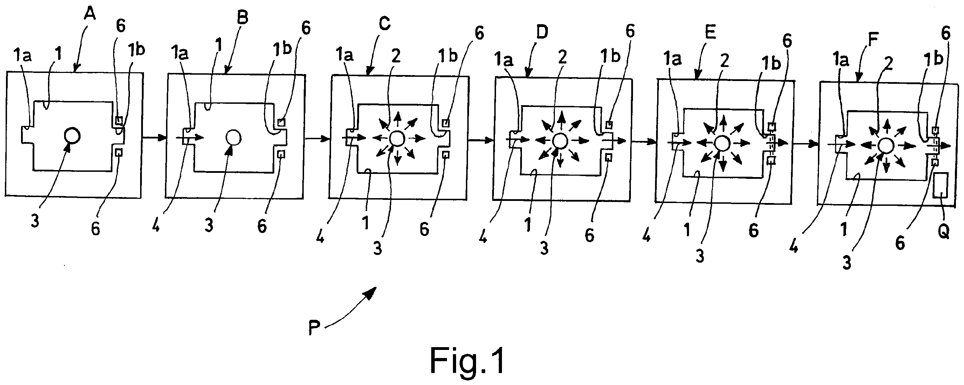

As can be seen in the block diagram represented in figure 1, a process P is schematically shown, for detecting the concentration and/or amount of carbon dioxide released by a patient.

The process P comprises a provision phase A of at least one chamber 1 provided with an inlet 1a and an outlet 1b, suitable for receiving in its interior, the carbon dioxide 2 released by a patient 3 affected by respiratory problems.

This is followed by a phase B for the continuous entry, through the inlet 1a, of a predetermined amount of a first fluid 4 which comprises a predetermined percentage and/or amount of gaseous mixture whose composition is established at the beginning of the process in relation to the pathology in question and conditions of the patient.

More specifically, the first fluid 4 is composed of a mixture of gases which comprises at least a predetermined percentage and/or amount of oxygen and a known percentage and/or amount of carbon dioxide, preferably equal to or close to 0%.

Once the first fluid 4 has been introduced A into the above chamber 1, the amount and/or percentage of carbon dioxide 2 released by the patient 3 into the chamber 1 is at least partly mixed C with the first fluid 4 previously introduced.

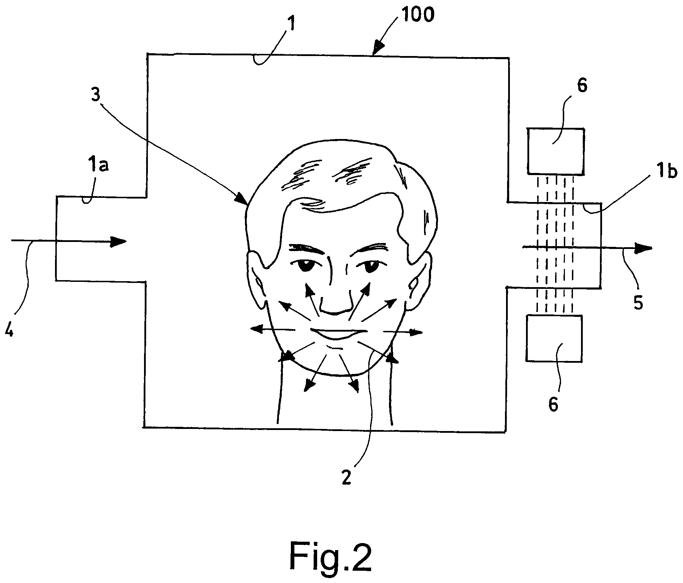

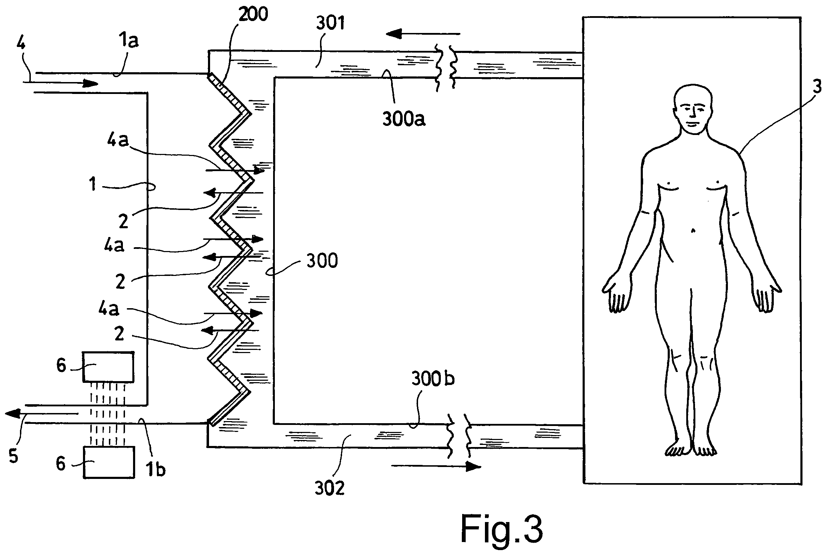

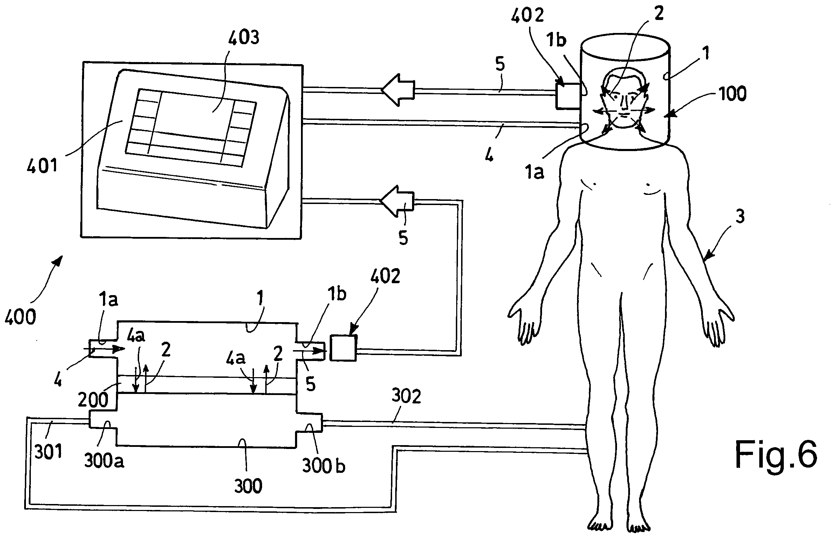

At least a part of the oxygen present in the first fluid 4 is naturally assimilated by the patient 3 through an exchange between oxygen and blood which can take place either directly in the pulmonary alveoli of the patient 3, as, for example, in NIMV (non-invasive ventilation) treatment (figures 2 and 6), or by means of suitable exchange membranes 200 (figures 3 and 6), such as, for example, those used in "EC" (ECMO/DECAP - extracorporeal oxygenation /decapneization) treatment.

The mixture of the first fluid 4 with carbon dioxide 2 released by the patient 3 defines a second fluid 5 different from the first fluid 4, as it has a greater percentage and/or amount of carbon dioxide 2 with respect to the initial one and a lower percentage of oxygen with respect to the initial one.

The second fluid 5 is continuously discharged D through the outlet 1b of the chamber 1 used.

In this case, the second fluid 5 is subject to a detection phase E of the percentage and/or amount of carbon dioxide 2 present in the same.

On the basis of what emerges from the detection phase E, the amount and/or percentage Q of carbon dioxide 2 released by the patient 3 into the chamber 1 is then determined in a predetermined time interval, preferably equal to a minute.

According to a preferred aspect of the present invention, the amount Q of carbon dioxide 2 released by the patient 3 in the first fluid 4 is determined F by comparing the composition and/or amount, expressed in rate, of the latter with the composition and/or the amount, also expressed in rate, of the second fluid 5.

In this respect, it is particularly advantageous to measure the rate of the flow of the first fluid 4 entering the chamber 1 and/or of the second fluid 5 leaving the same chamber using at least one respective flow meter.

More specifically, the amount of the first fluid 4 introduced into the chamber 1 substantially corresponds to the amount of second fluid 5 discharged from the same chamber, consequently the detection E of the concentration and/or amount of carbon dioxide 2 released by the patient 3 in the first fluid 4 is effected and/or determined F by directly detecting the amount and/or percentage of carbon dioxide 2 in the second fluid 5 discharged D, which was not present in the first fluid 4.

Consequently, a comparison between the first fluid 4 and the second fluid 5 reveals the amount Q of carbon dioxide 2 released by the patient 3 into the chamber 1 and present in the second fluid 5, and also the amount of the first fluid 4 administered to, or assimilated by, the patient 3.

Advantageously the detection E of the concentration and/or amount of carbon dioxide 2 in the second fluid 5 is effected periodically, optionally at regular time intervals. There is consequently a continuous control of the amount and/or percentage Q of carbon dioxide 2 eliminated by the patient 3 during a predetermined period of time.

According to a further advantageous aspect of the present invention, the detection E of the concentration and/or amount of carbon dioxide 2 present in the second fluid 5 is effected using one or more infrared-ray detectors 6 operatively positioned in correspondence with the outlet 1b of the chamber 1, i.e. in correspondence with the discharge or downstream of the same.

The detection E of the concentration and/or amount of carbon dioxide 2 present in the second fluid 5 is advantageously carried out in parts per million. The parts per million can be transformed, by means of appropriate specific algorithms, into a flow, expressed for example in millimeters or liters per minute, and/or into a pressure, expressed for example in millimeters of mercury.

According to a preferred aspect of the invention, if the flow and composition of the first fluid 4 at the inlet of the chamber 1 and the flow and composition of the second fluid 5 at the outlet of the same, are known, the detection phase E allows the total amount Q of carbon dioxide 2 released from the patient's 3 body in a minute, to be measured.

With particular reference to NIMV treatment, the process according to the present invention envisages that the chamber 1 with the inlet 1a and outlet 1b be defined by a respiration interface 100, such as, for example, a helmet, a mask or any other medical device which allows the natural exchange of carbon dioxide 2 and oxygen, in a controlled atmosphere.

As can be seen in figure 2, the chamber 1 is preferably defined inside a respiration interface 100 for artificial and/or assisted breathing.

In this case, the first fluid 4, containing a known percentage and/or amount of oxygen, is continuously introduced B into the respiration interface 100 according to an ingoing flow of about 30-80 litres per minute.

It is not excluded that the first fluid 4 be continuously introduced B into the respiration interface 100 according to an ingoing flow of gas of about 30-120 litres per minute.

The exhalation of the patient 3 causes the same to release an amount and/or percentage of carbon dioxide 2 into the first fluid 4 introduced B into the respiration interface 100.

The carbon dioxide 2 exhaled by the patient 3 is diluted in the first fluid 4, defining a second different fluid 5 which is consequently discharged D, through the outlet 1b.

The second fluid 5 is advantageously discharged D from the respiration interface 100 through the outlet 1b with a discharge flow of about 30-80 litres per minute, that is to say substantially at the same rate as the feeding.

If the ingoing rate of the first flow of gas of the first fluid 4 is about 30-120 litres per minute, the rate of the second flow of gas of the second fluid 5 is also about 30-120 litres per minute.

The discharge flow of the second fluid 5 is passed through the infrared detector 6 which reveals its concentration and/or amount of carbon dioxide 2 present therein, at close or continuous time intervals.

By measuring the concentration and/or amount of carbon dioxide 2 revealed in the discharge flow of the second fluid 5, and if the amount and composition of the fluids at the inlet and outlet are known, the total amount of carbon dioxide 2 exhaled in a minute by the patient 3, can be determined. In other words, as the amount and/or composition of the ingoing flow of gases and the amount and composition of the outgoing flow of gases, as well as the actual amount and/or concentration, expressed in parts per million, of carbon dioxide 2 in the outgoing flow of gases are known, it is possible, also by a comparison between said flows, to determine the actual total amount Q of carbon dioxide 2 produced by the patient 3, which is advantageously expressed in milliliters or liters per minute.

With particular reference to extracorporeal circulation "EC" treatment ECMO/DECAP, the process P according to the present invention envisages that the chamber 1 with the inlet 1a and outlet 1b be defined in correspondence with an exchange membrane 200 between oxygen 4a and carbon dioxide 2, as illustrated in figure 3.

In this case, the first fluid 4, containing a known percentage of oxygen, is introduced B into the chamber 1 according to an ingoing flow ranging from 2 to 12 liters per minute.

It is not excluded that the first fluid 4 be introduced B into the chamber 1 through an ingoing flow of gas ranging from 0.1 to 12 liters per minute.

As it can be seen in figure 3, at least a part of the chamber 1 is delimited by the exchange membrane 200 whose structure is permeable to gases so as to allow a two-directional passage of the latter.

On the opposite side of the chamber 1, the exchange membrane 200 partially delimits an auxiliary chamber 300 also provided with an inlet 300a and an outlet 300b. The auxiliary chamber 300, as also the inlet 300a of the latter, is occupied by the venous blood 301 of the patient 3 full of carbon dioxide 2.

The partial pressure difference between the chamber 1 and the auxiliary chamber 300 causes an exchange of gases between the first fluid 4 and the venous blood 301 of the patient 3, and consequently a part of the oxygen 4a present in the first fluid 4 passes, through the porosity of the membrane 200, from the chamber 1 to the auxiliary chamber 300, whereas the carbon dioxide 2 present in the venous blood 301, passes from the auxiliary chamber 300 to the chamber 1, still through the porosity of the membrane 200. Due to this exchange of gas, the venous blood 301 is oxygenated before being expelled from the auxiliary chamber 300. The oxygenated blood 302 is then directed, through the outlet 300b, towards the patient 3 undergoing treatment.

At least part of the carbon dioxide 2 eliminated by the patient 3 through the membrane 200 is contemporaneously mixed with the remaining gases of the first fluid 4 transforming it into the second fluid 5 to be discharged D through the outlet 1b.

The second fluid 5 is discharged D through the outlet 1b and the detector 6, which reveals 5 the actual concentration and/or amount of carbon dioxide 2 eliminated from the patient's 3 blood.

Also in this situation, if the amount and composition of the flow of the first fluid 4 at the inlet 1a, the flow of the second fluid 5 at the outlet 1b and the concentration and/or amount of carbon dioxide 2 present in the latter, are known, the amount Q of carbon dioxide 2 eliminated from the patient's 3 body can be determined within a predetermined time range, preferably about a minute.

An object of the present invention also relates to an apparatus 400 for the real time and high reliability detection of the actual concentration and/or total amount Q of carbon dioxide 2 released C by a patient 3 in a flow of gas to be monitored containing carbon dioxide at the inlet of a chamber 1, indicated hereunder with the letter "X".

In particular, the chamber 1 is provided with an inlet 1a for the entry of a first flow of gas 4 having a known concentration and/or amount of carbon dioxide 2 and an outlet 1b for the outlet of a second flow of gas 5 given by the mixing of said flow of gas to be monitored X with the first flow of gas 4.

The apparatus 400 also comprises detecting means 402 suitable for being associated with said chamber 1. The detecting means 402 comprise at least one detector 6 for the continuous detection and at regular time intervals, of the actual concentration and/or amount of carbon dioxide 2 contained in the second flow of gas 5.

The detector 6 is preferably any detector of the infrared optical type.

In the embodiment illustrated in figure 2, the detector 6 is applied on or at the wall(s) of the outlet duct 1b of the chamber 1 so that the beam of infrared rays generated thereby is traversed by the second flow/fluid of gas 5.

It should be pointed out, however, that infrared optical detectors different from that illustrated can also be used, such as, for example, optical detectors positioned inside the outlet duct 1b of the chamber 1 to remain immersed in the second flow/fluid of gas 5 leaving the latter.

The detector 6 is preferably operatively positioned at the outlet 1b of the chamber 1 or downstream thereof, immersed in the flow of gas 5, for measuring the acgtual concentration and/or amount of carbon dioxide contained in the second flow of gas 5.

The apparatus 400 advantageously also comprises a measuring device or detector of the rate/flow of the first and/or second flow of gas 4, 5.

In addition, the apparatus can also comprise a further detector 6 of the concentration and/or amount of carbon dioxide 2 contained in the first flow of gas 4.

In order to continuously determine of F the concentration and/or amount of Q of carbon dioxide 2 per unit of time of the gas flow to be monitored X, the apparatus 400 is advantageously equipped with at least one programmable electronic unit 401.

In order to visualize the data revealed E and/or determined F by means of the detectors and the programmable electronic unit 401, respectively, the apparatus 400 is advantageously equipped with at least one display 403 for the continuous visualization and in real time of the amount Q of carbon dioxide 2 per unit of time of the gas flow to be monitored X.

With particular reference to the solutions of figures 2 and 3, the flow of gas to be monitored X, containing carbon dioxide 2, is released C inside the chamber 1 by a patient 3 subjected to a treatment, in particular NIMV or extracorporeal circulation "EC" treatment, of the ECMO/DECAP type.

If the treatment to which the patient 3 is subjected is of the NIMV type, i.e. treatment for artificial and/or assisted respiration in a "non-invasive" mode, a helmet is used as respiration interface 100, or a mask or any other analogous medical device, wherein the gas flow to be monitored X consists of the release C of carbon dioxide 2 of the patient 3 into the chamber 1 through the expiratory act effected during the respiration of the same.

In this situation, the first flow of gas 4 is continuously introduced B into the respiration interface 100 at about 30-80 litres per minute or about 30-120 litres per minute.

The second gas flow 5 is preferably discharged D from the respiration interface 100 at about 30-80 litres per minute or about 30-120 litres per minute that is to say substantially at the same rate as the feeding.

With reference, on the contrary, to extracorporeal oxygenation treatment "EC", i.e. treatment of the ECMO/DECAP type, the chamber 1 is defined in correspondence with a side of a gaseous exchange membrane 200 which is in contact on the opposite side with respect to the chamber 1, with the venous blood of the patient 3.

In order to allow the two-directional passage of oxygen and carbon dioxide 2 from the chamber 1 towards the patient's 3 blood and vice-versa, the structure of the gaseous exchange membrane is permeable to gases. In this case, the flow to be monitored X consists of the release C of carbon dioxide 2 from the patient 3 into the chamber 1 through the gaseous exchange membrane 200.

In this situation, the first flow of gas 4, is continuously introduced B into the chamber 1, at about 2-12 litres per minute or at about 0.1-12 litres per minute, in correspondence with the gaseous exchange membrane 200. The second flow of gas 5 is also continuously discharged D from the chamber 1 at about 2-12 litres per minute or at about 0.1-12 litres per minute, that is to say substantially at the same rate as the feeding.

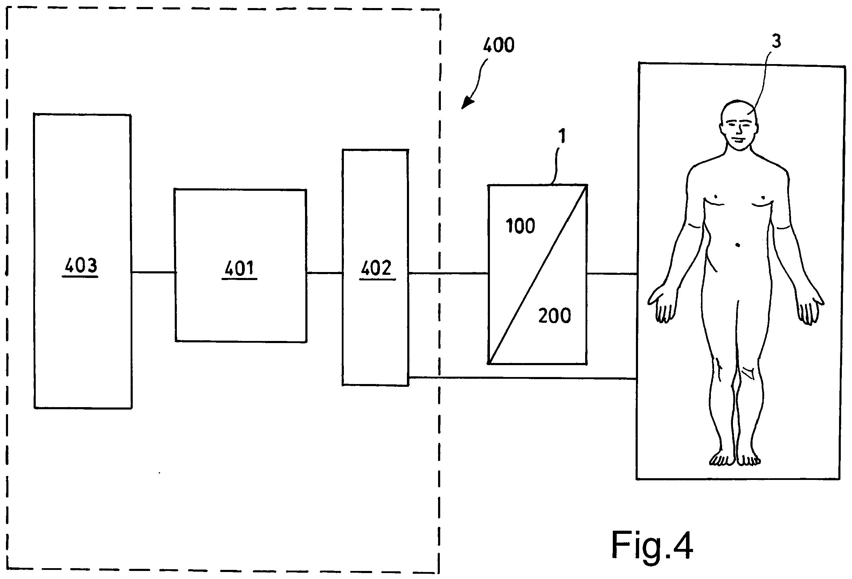

More specifically, as can be seen in the block scheme of figure 4, the apparatus 400 comprises at least one programmable electronic unit 401, equipped with at least one microprocessor or similar data processor and with an appropriate inner circuitry (not shown in the figures). The programmable electronic unit 401 is advantageously equipped with suitable detection means 402 suitable for revealing a series of parameters and data relating to both the patient 3 undergoing treatment, such as, for example, the physiological parameters of the same, and also to the exchange between oxygen and carbon dioxide 2, which takes place naturally during NIMV assisted respiration treatment or artificially by means of the medical devices used in extracorporeal circulation "EC" treatment and in ECMO/DECAP treatment, such as exchange membranes.

The detection means 402 are advantageously operatively connected to chamber 1, to reveal, in correspondence with the outlet 1b or downstream of the same, the actual concentration and/or total amount of carbon dioxide 2 released by the patient 3 in the chamber 1 per unit of time.

Again with reference to the block scheme of figure 4, the apparatus 400 is also equipped with the display 403 or another similar visualization interface suitable for visualizing, even contemporaneously, all the parameters revealed during the treatments of the patients 3, and also the parameters obtained by implementing the corresponding transformation algorithms, previously envisaged in the apparatus 400.

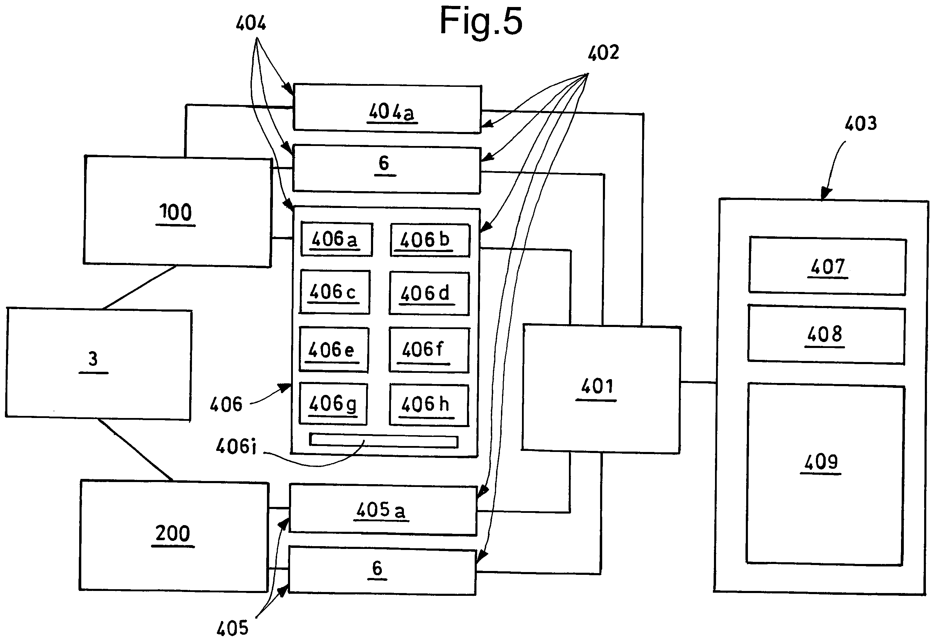

More specifically, as illustrated in the block scheme of figure 5, the detection means 402 comprise a first group of detectors 404 specific for the respiration interfaces 100, of the type used in NIMV assisted respiration treatment, and a second group 405, specific for the exchange membranes 200 used in ECMO/DECAP treatments and in extracorporeal circulations.

The first group 404 is equipped with at least one flow detector 404a for detecting the rate of at least one gas flow and at least one detector 6 of the concentration and/or amount of carbon dioxide 2 present in a flow of gas.

Both the flow detector 404a and the detector 6 of the concentration and/or amount of carbon dioxide 2 are preferably positioned so that can operate directly on the flow of the second fluid 5 at the outlet 1b of the chamber 1.

With reference to the interface 100, the above-mentioned detectors 6, 404a (figure 6) can be advantageously directly engaged at the outlet duct of the interface 100 or at the respective duct which extends from the same.

The first group 404 of detecting means 402 advantageously comprises at least one subgroup 406 which optionally envisages at least one multiparameter sensor or a plurality of sensors assigned for detecting various parameters.

More specifically, the subgroup 406 is provided with: a first detection unit 406a of the oxygen saturation in the arterial blood of the patient 3 SpO₂; a second detection unit 406b of the patient's 3 pulse; a third unit 406c for monitoring the positive pressure in a CPAP apparatus; a fourth detection unit 406d of the fraction of oxygen inhaled FiO₂; a fifth detection unit 406e of the patient's 3 temperature; a sixth detection unit 406f of the relative humidity; a seventh detection unit 406g of the absolute humidity; an eighth detection unit 406h of the respiratory frequency of the patient 3, a ninth detection unit 406i of the amount of fluid 4 introduced into the chamber 1, a particularly innovative element when the fluid generator 4, is a Venturi-type generator.

Analogously to the first group 404, the second group 405 comprises at least one flow detector 405a for detecting the rate of at least one flow of gas and at least one detector 6 of the concentration and/or amount of carbon dioxide 2 present in a flow of gas.

The flow detectors 404a, 405a and the detectors of the concentration and/or amount of carbon dioxide 2 of both groups 404 and 405 are respectively similar, as they are designed for substantially revealing the same datum. The only detectable difference between these detectors lies in the scale and in the instrumental sensitivity, which must obviously change in correspondence with the different rates envisaged in both NIMV/CPAP treatment and in "EC" (ECMO/DECAP) treatment.

With reference to the membrane 200 used in "EC" treatment (ECMO/DECAP), the above-mentioned detectors 6, 405a can be advantageously engaged directly at the discharge or outlet 1b of the chamber 1 or at the respective duct which extends from the same.

The display 403 advantageously comprises: a first panel or dial 407 for visualizing the values of the concentration and/or amount of carbon dioxide 2 eliminated with time VeCO₂ by the patient 3, if subjected to NIMV treatment, a second panel or dial 408 for visualizing the values of the concentration and/or amount of carbon dioxide 2 eliminated VCO₂ by the patient 3, if subjected to "EC" treatment (ECMO/DECAP); a third panel or dial 409 in which the values revealed by the detectors 406a-406g are grouped.

With reference to the process P described above, the provision phases A of the chamber 1, introduction B of the first fluid 4, releasing C of carbon dioxide 2 into the chamber 1, discharging D of the second fluid 5 from the chamber 1, prevalently affect the chamber 1 defined in the interface 100 or in correspondence with the exchange membrane 200.

The detection phase E involves both the detectors 6 and also the programmable electronic unit 401 of the apparatus 400 which receives the data revealed by the latter.

The determination phase F of the amount Q of carbon dioxide 2 eliminated by the patient 3 over a period of time, exclusively involves the programmable electronic unit 401, whereas the comparison between the first fluid 4 and the second fluid 6 necessary for determining F the actual amount Q of carbon dioxide 2 eliminated by the patient 3 during the treatment, is effected by the programmable electronic unit 401 and is implemented by a proper software or similar program preferably loaded in the same or accessible thereto.

The process and apparatus described above solve the problems encountered in the known art and obtain important advantages.

First of all, the process and apparatus described allow the continuous and real-time detection of the actual concentration and/or amount of carbon dioxide, total and not sampled, released by the patient or eliminated by the same in a predetermined unit of time during both NIMV treatment and also during "EC" (ECMO/DECAP) treatment.

With particular reference to NIMV treatment, when both the amount and composition of the flow of gases at the inlet and outlet into/from the respiration interface, are known, the detection of the concentration and/or amount of carbon dioxide released by the patient inside the respiration interface allows the total amount of carbon dioxide exhaled VeCO₂ by the patient to be determined in a predetermined time interval, consequently having an immediate indication of the respiratory, physiological and metabolic conditions of the same.

Furthermore, the precision and immediacy of the measurement of carbon dioxide eliminated from the patient's body VeCO₂ allow a significant reduction or even elimination of blood gas analysis which is effected exclusively for revealing important parameters and/or values for the doctors.

It should also be pointed out that the monitoring of patients subjected to NIMV treatment allows various critical situations to be predicted and anticipated, ensuring the patient's safety.

More specifically, the above monitoring of the VeCO₂ provides precise and objective information on the physiological variables and consequently, on the state of the patients.

The measurement of the VeCO₂ obtained according to the present process also advantageously allows other important values for the care treatment to be carried out on the patients, to be accurately obtained.

In particular, the respiratory metabolism, the cardiac output, the alveolar dead space, as well as the quantification of calories, can also be easily effected without having to resort to complex calculations and starting from measurable parameters.

In addition, the process described above allows NIMV therapies to be managed in the best possible way, with the continuous monitoring of the carbon dioxide VeCO₂ produced and eliminated by patients affected by Acute Respiratory Insufficiency, of both a hypoxemic and also hypercapnic nature, without the help of EGA.

With particular reference to "EC" (ECMO/DECAP) treatment, the detection of the amount of carbon dioxide eliminated VCO₂ following the gaseous exchange that takes place in correspondence with the exchange membrane, allows the amount of carbon dioxide which passes from the venous blood through the exchange membrane to be determined during a predetermined time interval. This value is particularly important as it provides an immediate and continuous indication of the patient's state and/or effectiveness of the exchange membrane.

Any loss in the effectiveness of the exchange membrane, in fact, causes a significant variation in the measurement of the concentration and/or amount of carbon dioxide eliminated. This variation, if not due to a worsening in the patient's conditions, determines the immediate substitution of the exchange membrane used with a new more effective exchange membrane.

The process and apparatus described above also allow, during both NIMV treatment and "EC" (ECMO/DECAP) treatment, an immediate and abrupt setting of the parameters and definitions relating to the functioning of the devices and/or systems used, in response to the variations revealed in the concentration and/or amount of carbon dioxide eliminated.

## Claims

1. An apparatus (400) comprising a chamber (1) adapted to contain at least a part of a patient (3), said chamber (1) comprising an inlet (1a) for a first flow of gas (4) comprising carbon dioxide (2), and an outlet (1b) for a second flow of gas (5) comprising carbon dioxide (2),
detecting means (402) suitable for being associated with said outlet (1b) of said chamber (1), said detecting means (402) comprising a first and a second detector (6) for a continuous detection at regular time intervals of a respective first and a second concentration of carbon dioxide (2) of said first flow of gas (4) and said second flow of gas (5), said first and second detector (6) being operatively positioned, respectively, at the inlet (1a) and at the outlet (1b) of the chamber (1) so that the total flow of the first (4) and second flows of gas (5) passes through the respective detector (6),
at least one measuring device suitable for measuring a first flow rate of said first flow of gas (4) and a second flow a rate of said second flow of gas (5),
at least one programmable electronic unit (401) configured to calculate a first total amount of carbon dioxide (2) of the first flow of gas (4) by multiplying the first concentration with the first flow rate and to calculate a second total amount of carbon dioxide (2) of the second flow of gas (5) by multiplying the second concentration with the second flow rate;
said at least one programmable electronic unit (401) is further configure to determine (F) continuously a third total amount (Q) of carbon dioxide (2) per unit of time of a third flow of gas by subtracting the second total amount of carbon dioxide (2) from the first total amount of carbon dioxide (2), wherein said third flow of gas is released by the patient (3) into said chamber (1) .

2. Apparatus (400) according to claim 1, comprising at least one display screen (403) suitable for displaying continuously and in real time the third total concentration and/or the third total amount (Q) of carbon dioxide (2) per unit of time of said third flow of gas to be monitored (X).

3. Apparatus (400) according to anyone of the claims 1 or 2, wherein said chamber (1) is a respiration interface (100) suitable for artificial respiration and/or assisted respiration, such as a helmet, a mask or any other similar medical device, wherein said third flow of gas to be monitored (X) consists of a release (C) of carbon dioxide (2) by the patient (3) inside said chamber (1) by the expiration during the respiration thereof.

4. Apparatus (400) according to claim 3, wherein said respiration interface (100) is adapted to fed (B) continuously said first flow of gas (4) at a rate of 30-80 liters per minute or at a rate of 30-120 liters per minute and/or said respiration interface (100) is adapted to discharge (D) continuously said second flow of gas (5) at a rate of 30-80 liters per minute or at 30-120 liters per minute, that is to say substantially at the same rate as the feeding.

5. Apparatus (400) according to anyone of the claims 1 or 2, wherein said chamber (1) is defined at a side of a gas exchange membrane (200), said gas exchange membrane (200) being in contact on the opposite side with respect to said chamber (1) and is adapted for being in contact with venous blood of said patient (3), said gas exchange membrane (200) comprises a structure being permeable to gases, said third flow to be monitored (X) consisting of the release (C) of said carbon dioxide (2) from the patient (3) through said gas exchange membrane (200).

6. Apparatus (400) according to claim 5, wherein said chamber (1) is adapted to fed (B) continuously said first flow of gas (4) at a rate of 2-12 liters per minute or at a rate of 0.1-12 liters per minute, and/or said chamber (1) is adapted to discharge (D) continuously said second flow of gas (4) at a rate of 2-12 liters per minute or at a rate of 0.1-12 liters per minute, that is to say substantially at the same rate as the feeding.

7. Apparatus (400) according to anyone of the claims 1-6, wherein each detector (6) comprises one or more optical detectors (6).

8. Apparatus (400) according to claim 7, wherein said one or more optical detectors (6) are infrared detectors.

9. Apparatus (400) according to anyone of the claims 1-8, wherein each detector (6) is suitable to measure the concentration of carbon dioxide in the flow of gas (4, 5) at a frequency comprised between 1 Hz and 120 Hz, preferably between 80 Hz and 120 Hz, most preferably 100 Hz.

10. Apparatus (400) according to anyone of the claims 1-9, wherein said at least one measuring device suitable for measuring the rate of said first flow of gas (4) and the rate of said second flow of gas (5) comprises at least two flow meter.

## Patentansprüche

1. Vorrichtung (400), umfassend
eine Kammer (1), die dazu geeignet ist, mindestens einen Teil eines Patienten (3) aufzunehmen, wobei die Kammer (1) einen Einlass (1a) für einen ersten Gasstrom (4) umfassend Kohlendioxid (2) umfasst, und einen Auslass (1b) für einen zweiten Gasstrom (5), umfassend Kohlendioxid (2),
Bestimmungsmittel (402), die dazu geeignet sind, mit dem Auslass (1b) der Kammer (1) verbunden zu werden, wobei die Bestimmungsmittel (402) einen ersten und einen zweiten Detektor (6) umfassen, um in regelmäßigen Zeitintervallen eine jeweilige erste und eine zweite Konzentration von Kohlendioxid (2) des ersten Gasstroms (4) und des zweiten Gasstroms (5) zu bestimmen, wobei die ersten und zweiten Detektoren (6) jeweils an dem Einlass (1a) und an dem Auslass (1b) der Kammer (1) wirkpositioniert sind, so dass der Gesamtstrom der ersten (4) und zweiten Gasströme (5) durch den jeweiligen Detektor (6) hindurchgeht,
mindestens eine Messvorrichtung, die dazu geeignet ist, eine erste Durchflussmenge des ersten Gasstroms (4) und eine zweite Durchflussmenge des zweiten Gasstroms (5) zu messen,
mindestens eine programmierbare elektronische Einheit (401), die ausgebildet ist, um eine erste Gesamtmenge von Kohlendioxid (2) des ersten Gasstroms (4) durch Multiplizieren der ersten Konzentration mit der ersten Durchflussmenge zu berechnen und eine zweite Gesamtmenge von Kohlendioxid (2) des zweiten Gasstroms (5) durch Multiplizieren der zweiten Konzentration mit der zweiten Durchflussmenge zu berechnen;
wobei die mindestens eine programmierbare elektronische Einheit (401) ferner ausgebildet ist, um eine dritte Gesamtmenge (Q) von Kohlendioxid (2) pro Zeiteinheit eines dritten Gasstroms durch Subtrahieren der zweiten Gesamtmenge von Kohlendioxid (2) von der ersten Gesamtmenge von Kohlendioxid (2) zu bestimmen (F), wobei der dritte Gasstrom von dem Patienten (3) in die Kammer (1)freigesetzt wird.

2. Vorrichtung (400) nach Anspruch 1, umfassend mindestens einen Anzeigebildschirm (403), der dazu geeignet ist, kontinuierlich und in Echtzeit die dritte Konzentration und/oder die dritte Gesamtmenge (Q) von Kohlendioxid (2) pro Zeiteinheit des dritten zu überwachenden Gasstroms (X) anzuzeigen.

3. Vorrichtung (400) nach einem der Ansprüche 1 oder 2, wobei die Kammer (1) eine Beatmungsschnittstelle (100) ist, die für die künstliche Beatmung und/oder assistierte Beatmung geeignet ist, wie beispielsweise ein Helm, eine Maske oder jedes andere ähnliche Medizinprodukt, wobei der dritte zu überwachende Gasstrom (X) aus einer Freisetzung (C) von Kohlendioxid (2) durch den Patienten (3) innerhalb der Kammer (1) durch das Ausatmen während der Beatmung desselben besteht.

4. Vorrichtung (400) nach Anspruch 3, wobei die Beatmungsschnittstelle (100) angepasst ist, um den ersten Gasstrom (4) kontinuierlich in einer Menge von 30-80 Liter pro Minute oder einer Menge von 30-120 Liter pro Minute einzuspeisen und/oder wobei die Beatmungsschnittstelle (100) angepasst ist, um kontinuierlich den zweiten Gasstrom (5) in einer Menge von 30-80 Liter pro Minute oder 30-120 Liter pro Minute, d. h. im Wesentlichen in der gleichen Menge wie beim Einspeisen abzuführen (D).

5. Vorrichtung (400) nach einem der Ansprüche 1 oder 2, wobei die Kammer (1) an einer Seite einer Gasaustauschmembran (200) definiert ist, wobei die Gasaustauschmembran (200) auf der gegenüberliegenden Seite in Bezug auf die Kammer (1) in Kontakt steht und angepasst ist, um mit venösem Blut des Patienten (3) in Kontakt zu stehen, wobei die Gasaustauschmembran (200) eine gasdurchlässige Struktur umfasst, wobei der dritte zu überwachende Strom (X) aus der Freisetzung (C) des Kohlendioxids (2) seitens des Patienten (3) durch die Gasaustauschmembran (200) besteht.

6. Vorrichtung (400) nach Anspruch 5, wobei die Kammer (1) angepasst ist, um den ersten Gasstrom (4) kontinuierlich in einer Menge von 2-12 Liter pro Minute oder einer Menge von 0,1-12 Liter pro Minute einzuspeisen (B) und/oder die Kammer (1) angepasst ist, um kontinuierlich den zweiten Gasstrom (4) in einer Menge von 2-12 Liter pro Minute oder einer Menge von 0,1-12 Liter pro Minute, d. h. im Wesentlichen in der gleichen Menge wie beim Einspeisen abzuführen (D).

7. Vorrichtung (400) nach einem der Ansprüche 1-6, wobei jeder Detektor (6) einen oder mehrere optische Detektoren (6) umfasst.

8. Vorrichtung (400) nach Anspruch 7, wobei der eine oder die mehreren optischen Detektoren (6) Infrarotdetektoren sind.

9. Vorrichtung (400) nach einem der Ansprüche 1-8, wobei jeder Detektor (6) dazu geeignet ist, die Konzentration von Kohlendioxid in dem Gasstrom (4, 5) bei einer Frequenz zwischen 1 Hz und 120 Hz, vorzugsweise zwischen 80 Hz und 120 Hz, besonders bevorzugt von 100 Hz zu messen.

10. Vorrichtung (400) nach einem der Ansprüche 1-9, wobei die mindestens eine Messvorrichtung dazu geeignet ist, die Durchflussmenge des ersten Gasstroms (4) und die Durchflussmenge des zweiten Gasstroms (5) zu messen, mindestens zwei Durchflussmesser umfasst.

## Revendications

1. Appareil (400) comprenant
une chambre (1) conçue pour contenir au moins une partie d'un patient (3), ladite chambre (1) comprenant une entrée (1a) pour un premier courant de gaz (4) comprenant du dioxyde de carbone (2), et une sortie (1b) pour un deuxième courant de gaz (5) comprenant du dioxyde de carbone (2),
des moyens de détection (402) appropriés pour être associés avec ladite sortie (1b) de ladite chambre (1), lesdits moyens de détection (402) comprenant un premier et un second détecteur (6) pour une détection continue à intervalles de temps réguliers d'une première et d'une seconde concentration de dioxyde de carbone (2) respectives dudit premier courant de gaz (4) et dudit deuxième courant de gaz (5), lesdits premier et second détecteurs (6) étant positionnés de manière fonctionnelle, respectivement, au niveau de l'entrée (1a) et au niveau de la sortie (1b) de la chambre (1) de sorte que le courant total des premier (4) et deuxième courants de gaz (5) traverse le détecteur respectif (6),
au moins un dispositif de mesure approprié pour mesurer un premier débit dudit premier courant de gaz (4) et un deuxième débit dudit deuxième courant flux de gaz (5),
au moins une unité électronique programmable (401) configurée pour calculer une première quantité totale de dioxyde de carbone (2) du premier courant de gaz (4) par la multiplication de la première concentration avec le premier débit et pour calculer une deuxième quantité totale de dioxyde de carbone (2) du deuxième courant de gaz (5) par la multiplication de la seconde concentration par le second débit ;
ladite au moins une unité électronique programmable (401) est en outre configurée pour déterminer (F) en continu une troisième quantité totale (Q) de dioxyde de carbone (2) par unité de temps d'un troisième courant de gaz par la soustraction de la deuxième quantité totale de carbone de dioxyde (2) à partir de la première quantité totale de dioxyde de carbone (2), dans lequel ledit troisième courant de gaz est libéré par le patient (3) dans ladite chambre (1).

2. Appareil (400) selon la revendication 1, comprenant au moins un écran d'affichage (403) approprié pour afficher en continu et en temps réel la troisième concentration totale et/ou la troisième quantité totale (Q) de dioxyde de carbone (2) par unité de temps dudit troisième courant de gaz à surveiller (X).

3. Appareil (400) selon l'une quelconque des revendications 1 ou 2, dans lequel ladite chambre (1) est une interface de respiration (100) appropriée pour la respiration artificielle et/ou la respiration assistée, telle qu'un casque, un masque ou tout autre dispositif médical similaire, dans lequel ledit troisième courant de gaz à surveiller (X) est constitué d'une libération (C) de dioxyde de carbone (2) par le patient (3) à l'intérieur de ladite chambre (1) par l'expiration au cours de sa respiration.

4. Appareil (400) selon la revendication 3, dans lequel ladite interface de respiration (100) est conçue pour introduire (B) en continu ledit premier courant de gaz (4) à un débit de 30 à 80 litres par minute ou à un débit de 30 à 120 litres par minute et/ou ladite interface de respiration (100) est conçue pour évacuer (D) en continu ledit deuxième courant de gaz (5) à un débit de 30 à 80 litres par minute ou à 30 à 120 litres par minute, c'est-à-dire à dire sensiblement au même débit que l'introduction.

5. Appareil (400) selon l'une quelconque des revendications 1 ou 2, dans lequel ladite chambre (1) est définie sur un côté d'une membrane d'échange de gaz (200), ladite membrane d'échange de gaz (200) étant en contact sur le côté opposé par rapport à ladite chambre (1) et est conçue pour être en contact avec le sang veineux dudit patient (3), ladite membrane d'échange de gaz (200) comprend une structure qui est perméable aux gaz, ledit troisième courant à surveiller (X) étant constitué de la libération (C) dudit dioxyde de carbone (2) par le patient (3) à travers ladite membrane d'échange de gaz (200) .

6. Appareil (400) selon la revendication 5, dans lequel ladite chambre (1) est conçue pour introduire (B) en continu ledit premier courant de gaz (4) à un débit de 2 à 12 litres par minute ou à un débit de 0,1 à 12 litres par minute et/ou ladite interface chambre (1) est conçue pour évacuer (D) en continu ledit deuxième courant de gaz (4) à un débit de 2 à 12 litres par minute ou à un débit de 0,1 à 12 litres par minute, c'est-à-dire à dire sensiblement au même débit que l'introduction.

7. Appareil (400) selon l'une quelconque des revendications 1 à 6, dans lequel chaque détecteur (6) comprend un ou plusieurs détecteurs optiques (6).

8. Appareil (400) selon la revendication 7, dans lequel lesdits un ou plusieurs détecteurs optiques (6) sont des détecteurs infrarouges.

9. Appareil (400) selon l'une quelconque des revendications 1 à 8, dans lequel chaque détecteur (6) est approprié pour mesurer la concentration de dioxyde de carbone dans le courant de gaz (4, 5) à une fréquence comprise entre 1 Hz et 120 Hz, de préférence entre 80 Hz et 120 Hz, idéalement de 100 Hz.

10. Appareil (400) selon l'une quelconque des revendications 1 à 9, dans lequel ledit au moins un dispositif de mesure approprié pour mesurer le débit dudit premier courant de gaz (4) et le débit dudit deuxième courant de gaz (5) comprend au moins deux débitmètres.
